# EUROPEAN PATENT SPECIFICATION

(11) **EP 2 185 120 B1**
(45) Date of publication and mention of the grant of the patent: **23.10.2013**
(21) Application number: 07794214.2
(22) Date of filing: 03.09.2007
(51) Int. Cl.: A61F 13/514, A61F 13/49, B32B 27/12

(54) **BACKSHEET FOR AN ABSORBENT ARTICLE**
RÜCKSEITE FÜR EINEN SAUGFÄHIGEN ARTIKEL
ENVELOPPE DE COUCHE POUR ARTICLE ABSORBANT

(43) Date of publication of application: 19.05.2010
(73) Proprietor: SCA Hygiene Products AB, 405 03 Göteborg (SE)
(72) Inventor: KROOK, Fredrik, SE-431 59 Mölndal (SE)
(74) Representative: Börlin, Maria
(86) International application number: PCT/SE2007/050609
(87) International publication number: WO 2009/031950

(56) References cited:
- EP-A1- 1 738 729
- WO-A1-99/60967
- WO-A1-99/60973
- WO-A2-2004/092470
- GB-A- 2 293 573
- US-A- 5 458 590
- US-A1- 2006 246 263

## Description

### TECHNICAL FIELD

The invention relates to a backsheet for an absorbent article comprising an inner liquid-impermeable film layer and an outer layer of nonwoven, said inner liquid-impermeable film layer including at least one printed area.

### BACKGROUND OF THE INVENTION

Diapers, training pants and the like absorbent articles have nowadays often a pant-like shape after being applied to a wearer and in order to give such articles an appearance more like a pair of underpants, the backsheet has often an outer layer having a textile-like appearance. In GB 2 293 573 A, which discloses a backsheet according to the first paragraph of the description, the appearance of a disposable absorbent garment has been improved by providing a film layer with a different colour than the outer layer of the backsheet and by providing transparent areas in the outer layer, in which the underlying film layer is visible. It is also known from this document to provide printed figures or drawings in the areas of the film layer covered by transparent areas of the outer layer. The transparent areas of the outer layer are manufactured by subjecting corresponding areas of an opaque nonwoven layer to heat and pressure so that the fibres in these areas melt and form a filmlike structure.

It is also known in the art to provide an outer nonwoven layer of a backsheet with a printed pattern. However, the quality of a printing on a nonwoven of the type used on absorbent articles is less distinct and does not allow printing of figures and drawings in which distinct lines or fully coloured areas are required in order to obtain the desired aesthetical effect.

The objective of the invention is to improve the versatility of printing on a backsheet without need to locally change the structure of the outer nonwoven layer of a backsheet.

### SUMMARY OF THE INVENTION

This objective is obtained by a backsheet for an absorbent article comprising an inner liquid-impermeable film layer and an outer layer of nonwoven, said inner liquid-impermeable film layer including at least one printed area, characterised in that said outer layer of nonwoven is provided with printing extending around but not into in an area or areas covering said at least one printed area of said inner liquid-impermeable film layer, the basis weight of said outer nonwoven layer being less than 20 g/m². Such a backsheet makes it possible to print a picture or a drawing which as a whole is covering the whole backsheet, distinct figures of said picture or drawing being printed on the inner liquid-impermeable film layer and the less distinct portions, e.g. the background of the picture or drawing, being printed on the outer nonwoven layer. Due to the low basis weight of the outer nonwoven layer, the print on the inner liquid-impermeable film layer will be visible to the viewer without noticeable obstruction. In contrast to the backsheet known from GB 2 293 573, the outer layer of a backsheet according to the present invention will have a textile structure over its whole area without interruption of film-like areas as is the case for the outer layer of the known backsheet. A backsheet according to the present invention can also be easily manufactured in a conventional process line for absorbent articles, the only additional equipment needed being printing equipment.

In a preferred embodiment, said inner liquid-impermeable film layer has two printed areas, one in the front portion and one in the rear portion, and the outer nonwoven layer has two unprinted areas corresponding in shape and location to the areas printed on the inner liquid-impermeable film layer. In a preferred variant, the inner liquid-impermeable film layer is only present in a middle part of the backsheet leaving the lateral side portion without film layer.

More than one printed area can, in a further preferred embodiment, be present on the front and/or rear portion of the backsheet.

It is possible but not preferred for the inner liquid-impermeable film layer or/and the outer nonwoven layer to be coloured.

Said outer nonwoven layer is preferably a spunbond or a melt blown and spunlaced nonwoven made from polypropylene fibres or blends of polyethylene and polypropylene and/or polyester fibres and said inner liquid-impermeable film is preferably comprised of polyethylene.

### BRIEF DESCRIPTION OF THE DRAWING

The invention will now be described with reference to the enclosed figures, of which;
Fig. 1 schematically discloses part of a web of a material for the inner liquid-impermeable film layer of a backsheet according to a first preferred embodiment of the invention,
Fig. 2 schematically discloses a web of a material for the outer nonwoven layer of a backsheet according to a first preferred embodiment of the invention, and
Fig. 3 illustrates the front portion of a diaper provided with a backsheet according to the first preferred embodiment of the invention, the diaper being put on a wearer.

### DESCRIPTION OF EMBODIMENTS

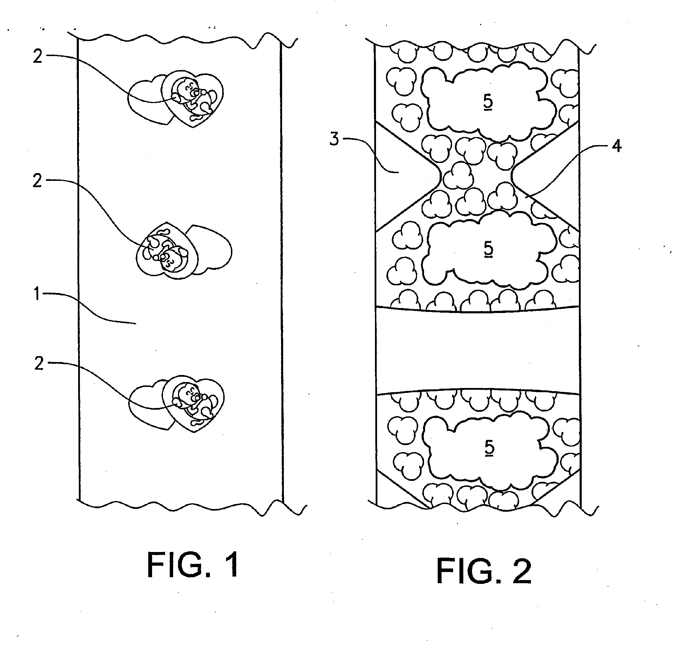

In Figure 1, a portion of a web 1 of a liquid impermeable material, e.g. polyethylene, is schematically disclosed. A row of printed areas 2 or pictures is printed on this web by suitable printing technique, for example by screen printing. Every second picture in said row is identical and intended to be located on the front or rear side of an absorbent article, such as a disposable diaper. In the shown embodiment adjacent pictures 2 in the row are identical but turned upside-down relative each other. The pictures 2 consist of two hearts, one heart overlapping the other, but other objects or figures can of course be used instead of hearts.

Figure 2 discloses a web 3 of nonwoven, e.g. a nonwoven of polypropylene fibres. A repeated pattern of hearts in a drawing 4 is printed on this web 3. This repeated pattern covers all areas of the web that will be present on backsheets of individual diapers after cutting of the web of backsheet material in one of the final steps of manufacturing of diapers, except for a first row of unprinted areas 5 and a second row of unprinted areas corresponding to the waist line regions of two adjacent blanks of articles to be cut from a composite web comprising webs 1 and 3, a web of top sheet material, a row of absorbent bodies disposed between webs 1,3 and the top sheet web, and elastic elements at least disposed in the waistline region. Each unprinted area 5 in the first row of unprinted areas corresponds to the area of a printed picture 2 on the web 1 of film material and is located at the same distance from each other and from the opposite edges of the web 3 as the pictures 2 in the row of pictures 2 on the web 1.

In order to create a web of backsheet material, the webs 1 and 3 are brought together and attached to each other. Web 3 is laid over web 1 so that the unprinted areas 5 on web 3 coincide with the pictures 2 on web 1. This is easily achieved if for example the webs are drawn from storage rollers and brought together and being attached to each other in the nip between two rolls simply by controlling that the first picture on web 1 coincide with the first unprinted area 5 on web 3. The webs 1 and 3 can preferably be glued together with a transparent glue or adhesive, for example hot melt adhesives. Other suitable means for attaching the webs together can also be used, for example ultra sonic welding or heat welding. The bringing together of the two webs 1 and 3 can be made in the production line for the manufacturing of absorbent articles or can be made beforehand, the web of backsheet material then being wounded on a storage roller to be used in said production line. The printing of the webs 1 and 3 can be made at any time and independently of each other, for example the printing of webs 1 and 3 can be made in connection with the process of bringing the webs together or the printing of web 1 can be made beforehand of the bringing together of the webs and the printing of web 3 can be made after the bringing together of the webs.

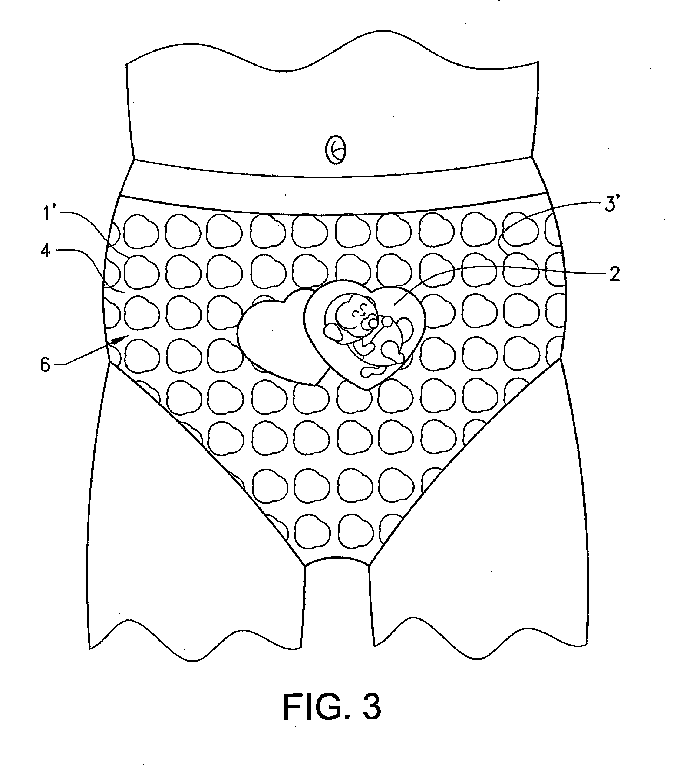

In Figure 3, a disposable article having a backsheet composed of material layers from webs 1 and 3 is shown in a schematic front view after being applied on a wearer. The absorbent article in Figure 3 is a training pant, i.e. a disposable article in which an individual article blank cut out of a composite web comprising a web of top sheet material, a web of backsheet material and a row of absorbent cores enclosed between said webs, is folded so that the ends of the front and rear portions of the cut out individual blank are aligned and the lateral side portions of the blank are attached to each other by a seam (not shown in the Figure). By front and rear portions is meant the front side and rear side of a training pant which is double folded with the free ends of the front and rear portions being aligned. The lateral sides of such a training pant are to the left and right in Figure 3, only the lateral sides of the front portion being visible in this Figure. In Figure 3, the layers of the backsheet of training pant 6 are given the same reference numerals as corresponding components in Figures 1 and 2 with the addition of a prime sign. As is evident from Figure 3, picture 2 on the inner film layer 1' fills up the whole unprinted area 5 of the outer nonwoven layer 3'. Thereby the viewer gets the impression of a homogenous picture instead of two different pictures or drawings as for an article according to GB 2 293 573.

In order to ascertain desired visibility of picture 2 through the unprinted area 5 on the nonwoven outer layer 3', the basis weight of layer 3' should be less than 20 g/m², preferably less than 18 g/m².

The advantage of using the inner film layer 1' for printing is that pictures or drawings with very distinct lines can be printed thereon. On the outer nonwoven layer 3', drawings or pictures will have blurred lines and indistinct areas due to the structure of this layer. If there is a need for sharp lines, such as a text line, the outer nonwoven layer 3' can therefore not be used.

In Figure 3 a figurative picture is printed on the backsheet. A backsheet according to the present invention can of course have other types of drawings or pictures printed thereon, for example abstract paintings or non-figurative patterns. More than one area on the front and/or rear portion of the backsheet could contain sharp lines or the like and be created by several printed areas on the inner film layer and corresponding unprinted areas on the outer nonwoven layer.

The inner layer 1' of the backsheet is preferably composed of polyethylene but can also consist of other polyolefins, such as polypropylene and polyester, and blends thereof. The inner layer 1' can also be breathable, i.e. having micropores over its surface.

The outer nonwoven layer 3' is preferably a spunbond, melt blown and spunlaced or carded nonwoven produced from fibres of polypropylene or other polyolefins. However any type of nonwoven material having a basis weight as stated above can be used. Layer 3' could also include several layers, such as a SMS (Spunbond-Meltblown-Spunbond)-composite material. Also natural fibres, such as e.g. cotton, starch and viscose fibres, can be used.

The backsheet according to the embodiment described can be modified in several respects without leaving the scope of invention. For example, the pattern or drawing on the outer nonwoven web could reach outside of the contour of the individual training pant blank to be cut out of this blank in order to ensure that no unprinted areas will be present on the ready made training pant. The pattern or drawing on the outer nonwoven web can be smaller than the contour of the individual training pant blank as long as it extends around the figure on the inner film sheet so that the viewer will perceive the different printings on the inner film layer and the outer nonwoven layer as one homogenous printing. The inner liquid impermeable film layer does not need to have the same dimensions as the outer nonwoven layer but could, for example, only be present in a middle part of the backsheet extending longitudinally from the front waist portion to the rear waist portion, thereby leaving the lateral side portions free of film material. It is not necessary to have the same pictures or drawings in the row or rows of figures printed on the inner film layer but figures of different shapes can be used as long as the unprinted areas on the outer nonwoven layer correspond in shape and location to said row or rows of different figures. More than one printed area can be present on the front and/or rear portion of the backsheet. Other printing techniques than screen printing can be used to print the inner and outer layer and different techniques can be used for the different layers. It is also possible that the inner film layer or/and the outer nonwoven layer can be coloured, i.e. the printing occurs on already coloured layer(s). Accordingly, this invention is intended to embrace all such alternatives, modifications and variations which fall within the scope of the enclosed patent claims.

## Claims

1. A backsheet for an absorbent article comprising an inner liquid-impermeable film layer (1') and an outer layer (3') of nonwoven, said inner liquid-impermeable film layer including at least one printed area (2), **characterised in that** said outer layer of nonwoven (3') is provided with printing (4) extending around but not into in an area or areas (5) covering said at least one printed area (2) of said inner liquid-impermeable film layer, the basis weight of said outer nonwoven layer being less than 20 g/m².

2. The backsheet according to claim 1, wherein said inner liquid-impermeable film layer (1') has two printed areas (2), one in the front portion and one in the rear portion, and the outer nonwoven layer (3') has two unprinted areas (5) corresponding in shape and location to the areas (2) printed on the inner liquid-impermeable film layer.

3. The backsheet according to claim 1 or 2, wherein the inner liquid-impermeable film layer is only present in a middle part of the backsheet leaving the lateral side portion without film layer.

4. The backsheet according to any one of claims 1-3, wherein more than one printed area is present on the front and/or rear portion of the backsheet.

5. The backsheet according to any one of claims 1-4, wherein the inner film layer or/and the outer nonwoven layer is coloured.

6. The backsheet according to any one of claims 1-5, wherein said outer nonwoven layer is a spunbond nonwoven.

7. The backsheet according to any one of claims 1-5, wherein said outer nonwoven layer is a melt blown and spunlaced or carded nonwoven.

8. The backsheet according to claim 6 or 7, wherein said nonwoven is made from polypropylene, polyethylene, polyethyleneterephthalate fibres or bicomponent fibres thereof, or fibres of polyamide or polylactic acid.

9. A backsheet according to any one of claims 1-8, wherein said inner liquid-impermeable film is comprised of polyethylene, other polyolefins, such as polypropylene and polyester, or blends thereof.

## Patentansprüche

1. Rückseite für einen absorbierenden Artikel, umfassend eine flüssigkeits-undurchlässige Filminnenschicht (1') und eine Außenschicht (3') aus Vliesmaterial, wobei die flüssigkeitsundurchlässige Innenfilmschicht mindestens einen bedruckten Bereich (2) aufweist, **dadurch gekennzeichnet, dass** die aus Vliesmaterial gefertigte Außenschicht (3') mit einem sich um einen Bereich bzw. um Bereiche (5) herum, aber nicht in einen Bereich bzw. in Bereiche (5) erstreckenden Druck (4) versehen ist, welcher Bereich bzw. welche Bereiche mindestens einen bedruckten Bereich (2) der flüssigkeitsundurchlässigen Innenfilmschicht überdeckt bzw. überdecken, wobei das Basisgewicht der aus Vliesmaterial gefertigen Außenschicht weniger als 20 g/m² beträgt.

2. Rückseite nach Anspruch 1, wobei die flüssigkeitsundurchlässige Innenfilmschicht (1') zwei bedruckte Bereiche (2) aufweist, einen in dem Frontteil und einen in dem Hinterteil, und die aus Vliesmaterial gefertigte Außenschicht (3') zwei unbedruckte Bereiche (5) aufweist, welche in Bezug auf Form und Position den auf der flüssigkeitsundurchlässigen Innenfilmschicht bedruckten Bereichen (2) entsprechen.

3. Rückseite nach Anspruch 1 oder 2, wobei die flüssigkeitsundurchlässige Innenfilmschicht nur in einem mittleren Teil der Rückseite vorgesehen ist, wobei das Seitenteil ohne Filmschicht vorliegt.

4. Rückseite nach einem der Ansprüche 1 bis 3, wobei mehr als ein bedruckter Bereich auf dem Frontteil und/oder dem Hinterteil der Rückseite vorgesehen ist.

5. Rückseite nach einem der Ansprüche 1 bis 4, wobei die Innenfilmschicht und/oder die aus Vliesmaterial gefertigte Außenschicht farbig ist bzw. sind.

6. Rückseite nach einem der Ansprüche 1 bis 5, wobei die aus Vliesmaterial gefertigte Außenschicht ein Spinnvlies ist.

7. Rückseite nach einem der Ansprüche 1 bis 5, wobei die aus Vliesmaterial gefertigte Außenschicht ein Meltblown- und Spunlace- oder kardiertes Vliesmaterial ist.

8. Rückseite nach Anspruch 6 oder 7, wobei das Vliesmaterial aus Polypropylen-, Polyethylen-, Polyethylenterephthalatfasern oder Zweikomponentenfasern davon, oder aus Polyamid- oder Polymilchsäurefasern hergestellt ist.

9. Rückseite nach einem der Ansprüche 1 bis 8, wobei die flüssigkeitsundurchlässige Innenfilmschicht Polyethylen oder Polyolefine, wie beispielsweise Polypropylen und Polyester, oder Gemische davon, umfasst.

## Revendications

1. Feuille de fond pour un article absorbant comprenant une couche de film intérieure imperméable aux liquides (1') et une couche extérieure (3') du non-tissé, ladite couche de film intérieure imperméable aux liquides comprenant au moins une zone imprimée (2), **caractérisée en ce que** ladite couche extérieure du non-tissé (3') est pourvue d'une impression (4) s'étendant autour de, mais non pas dans une ou plusieurs zones (5) recouvrant ladite au moins une zone imprimée (2) de ladite couche de film intérieure imperméable aux liquides, le poids de base de ladite couche extérieure du non-tissé étant inférieure à 20 g/m².

2. Feuille de fond selon la revendication 1, dans laquelle ladite couche de film intérieure imperméable aux liquides (1') présente deux zones imprimées (2), dont l'une pourvue dans la partie avant et l'une pourvue dans la partie arrière, et la couche extérieure du non-tissé (3') présente deux zones non imprimées (5) correspondant dans la forme et l'emplacement aux zones (2) imprimées sur la couche de film intérieure imperméable aux liquides.

3. Feuille de fond selon la revendication 1 ou 2, dans laquelle la couche de film intérieure imperméable aux liquides n'est présente que dans une partie médiane de la feuille de fond, laissant la partie de côté latéral sans une couche de film.

4. Feuille de fond selon l'une quelconque des revendications 1 à 3, dans laquelle plus d'une zone imprimée est présente sur la partie avant et/ou la partie arrière de la feuille de fond.

5. Feuille de fond selon l'une quelconque des revendications 1 à 4, dans laquelle la couche de film intérieure et/ou la couche extérieure du non-tissé est colorée.

6. Feuille de fond selon l'une quelconque des revendications 1 à 5, dans laquelle ladite couche extérieure du non-tissé est un non-tissé filé-lié.

7. Feuille de fond selon l'une quelconque des revendications 1 à 5, dans laquelle ladite couche extérieure du non-tissé est un non-tissé de fusion-soufflage et d'entrelacement hydraulique ou de cardage.

8. Feuille de fond selon la revendication 6 ou 7, dans laquelle ledit non-tissé est réalisé en polypropylène, polyéthylène, fibres de téréphtalate de polyéthylène ou fibres à deux composants de celui-ci, ou fibres de polyamide ou d'acide polylactique.

9. Feuille de fond selon l'une quelconque des revendications 1 à 8, dans laquelle ledit film intérieur imperméable au liquide est compris de polyéthylène, d'autres polyoléfines, telles que le polypropylène et le polyester, ou des mélanges de ceux-ci.
